(19) Europäisches Patentamt
European Patent Office
Office européen des brevets

(11) **EP 3 170 507 B1**

(12) **EUROPEAN PATENT SPECIFICATION**

(45) Date of publication and mention
of the grant of the patent:
**21.12.2022 Bulletin 2022/51**

(21) Application number: **15380049.5**

(22) Date of filing: **20.11.2015**

(51) International Patent Classification (IPC):
**A61K 38/08** *(2019.01)* **C07K 7/06** *(2006.01)*

(52) Cooperative Patent Classification (CPC):
**C07K 7/06; A23C 9/1315; A23C 9/1526;
A23J 1/006; A23L 2/02; A23L 33/18;** A61K 38/00

(54) **ANTIHYPERTENSIVE PEPTIDES FROM OLIVE OIL**

BLUTDRUCKSENKENDE PEPTIDE AUS OLIVENÖL

PEPTIDES ANTIHYPERTENSEURS FORMÉS À PARTIR D'HUILE D'OLIVE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**24.05.2017 Bulletin 2017/21**

(73) Proprietor: **Consejo Superior de Investigaciones
Científicas
(CSIC)
28006 Madrid (ES)**

(72) Inventors:
• **López-Huertas León, Eduardo
18008 Granada (ES)**
• **Alcaide Hidalgo, Juan Maria
18008 Granada (ES)**

(74) Representative: **Pons
Glorieta Rubén Darío 4
28010 Madrid (ES)**

(56) References cited:
**US-A- 4 477 440**

• **ESTEVE C ET AL: "Novel strategy for the
revalorization of olive (Olea europaea) residues
based on the extraction of bioactive peptides",
FOOD CHEMISTRY, vol. 167, January 2015
(2015-01), pages 272-280, XP002756686,**

• **SAADI SAMI ET AL: "Recent advances in food
biopeptides: Production, biological
functionalities and therapeutic applications",
BIOTECHNOLOGY ADVANCES, vol. 33, no. 1,
January 2015 (2015-01), pages 80-116,
XP002756687,**

• **MANDAL SANTI M ET AL: "Identification of
multifunctional peptides from human milk",
PEPTIDES, vol. 56, 2 April 2014 (2014-04-02),
pages 84-93, XP029032581, ISSN: 0196-9781,
DOI: 10.1016/J.PEPTIDES.2014.03.017**

• **Cristina Montealegre ET AL: "Separation of
proteins from olive oil by CE: An approximation
to the differentiation of monovarietal olive oils",
ELECTROPHORESIS, vol. 31, no. 13, 30 June
2010 (2010-06-30) , pages 2218-2225,
XP55586395, ISSN: 0173-0835, DOI:
10.1002/elps.200900675**

• **Álvaro Alonso ET AL: "Abstract", PUBLIC
HEALTH NUTRITION, vol. 9, no. 2, 1 April 2006
(2006-04-01), pages 251-257, XP55734003, GB
ISSN: 1368-9800, DOI: 10.1079/PHN2005836**

Remarks:
The complete document including Reference
Table(s) and the Sequence Listing(s) can be
downloaded from the EPO website

**Description**

The invention generally relates to a peptide isolated from olive oil, showing anti-hypertensive activity. The invention also relates to its use as a medicament for the treatment of pre-hypertension and hypertension.

**BACKGROUND OF THE INVENTION**

Hypertension, or high blood pressure, is a disease which affects approximately 40% of adults aged 25 and above. It is clinically defined as a systolic arterial blood pressure of 140 mm Hg or higher and a diastolic arterial blood pressure of 90 mm Hg or higher. Normal levels of both systolic and diastolic blood pressure are particularly important for the efficient function of vital organs such as the heart, brain and kidneys and for overall health and wellbeing. In addition, hypertension is a main risk factor for cardiovascular disease. It is a serious threat to the health of the population since in many cases it is the cause of coronary disease, stroke and myocardial infarction.

Although the causes of hypertension in most cases are unknown, the reninangiotensin system plays an important role in the regulation of blood pressure, blood volume and vascular tone. In this system, a peptide called angiotensin I is hydrolysed by the action of angiotensin converting enzyme (ACE). The reaction product, angiotensin II, is a potent vasoconstrictor. Angiotensin II induces hypertension by stimulating the contraction of smooth muscles in the walls of blood vessels. In addition, ACE stimulates the degradation of bradykinin, a peptide that reduces blood pressure. Therefore, inhibition of ACE prevents not only the formation of angiotensin II, stimulating peptide hypertension, but also inhibits the hydrolysis of peptides that lower blood pressure (bradykinin). Therefore, inhibition of ACE reduces blood pressure and hypertension can be reduced.

There are several pharmaceutical compounds on the market of which the mechanism of action is precisely the ACE inhibition. Examples of these drugs include benazepril (Lotensin), Captopril (Capoten), captopril / hydrochlorothizaide (Capozide), enalapril maleate (Vasotec), fosinopril (Monopril), lisinopril (Prinivil, Zestril), quinapril / magnesium carbonate (Accupril) , ramipril (Altace), trandolapril (Mavik). These ACE inhibitors are widely used in antihypertensive therapy and its use is continuously growing. Although these pharmaceuticals are effective in reducing blood pressure, there are numerous side effects produced by ACE inhibitors. They include the development of nocturnal dry cough, dizziness, headaches, and can cause allergic reactions that may occasionally be serious. ACE inhibitors can also cause potassium build-up and kidney problems, so potassium levels and kidney function should be monitored. Additionally, all of the ACE inhibitors appear to be capable of producing a severe allergic reaction that can be life-threatening.

Bioactive peptides ACE inhibitors have been isolated from different sources of animal and vegetable origin. The vast majority of ACE inhibitory peptides described so far, are obtained by the action of specific proteases on different sources of dietary proteins, including dairy (hydrolysed fractions of casein and whey) and fermented milks, eggs, soybeans, chickpeas, peanuts, tuna, sardines, shrimp, chicken, squid, among others [reviewed in Saadi S, Saari N, F Anwar Abdul Hamid A, HM Ghazali. Recent advances in food biopeptides: Production, biological functionalities and therapeutic applications. Biotechnol Adv. 2015, 33: 80-116]. The most studied and representative examples of these bioactive peptides ACE inhibitors are found in hydrolysates of milk proteins, carried out with different enzymes and by fermentation of milk with different bacteria. However, the existence of bioactive peptides with defined functions can also occur naturally (such as in breast milk) without the use of proteases for their production [Mandal SM, Bharti R, Porto WF, SS necessary Gauri , Mandal M, Franco OL, AK Ghosh. Identification of multifunctional peptides from human milk. Peptides. 2014, 56: 84-93]. The antihypertensive effects of some of these peptides derived from milk proteins have been studied in animal models and in human subjects [Ricci I Artacho R, M. Olalla With Milk protein peptides angiotensin I-converting enzyme inhibitory (ACEI) activity. Crit Rev Food Sci Nutr. 2010, 50:390-402]. Examples of peptides having antihypertensive activity demonstrated in animal models of hypertension and hypertensive humans are Ile-Pro-Pro (IPP) and Val-Pro-Pro (VPP). Likewise, the European patent applications EP1568707A1 and EP2495250A2 disclose novel antihypertensive peptides from casein hydrolysates.

The patent application US4477440A discloses peptides and analogs thereof which inhibit renin and are useful for treating various forms of renin-associated hypertension and hyperaldosteronism.

The publication of Esteve C. et al. 2015 (Food Chemistry, 167: 272-280) discloses anti-oxidant and anti-hypertensive peptides extracted from olive seeds hydrolysed with five different enzymes.

Nevertheless, it would be desirable to identify novel antihypertensive peptides alternative to those existing in the state of the art in order to improve the treatment of diseases associated with high blood pressure.

**DETAILED DESCRIPTION OF THE INVENTION**

The inventors of the present invention have discovered that peptides comprising two cysteines separated by 0 amino acids, consisting of a length between 8, 9 or 10 amino acids, and comprising the sequence SEQ ID NO: 7 are, surprisingly, capable of inhibiting the activity of the angiotensin converting enzyme (ACE), being thus useful as anti-hypertensive agents. The peptides were isolated and characterized from olive oil and by-products of the olive oil industry. As can be seen from Example 1, the raw materials were subjected to different processes of extraction with organic solvents and the extracts were concentrated to obtain a composition rich in peptides. These peptides were further purified using a fast system protein liquid chromatography (FPLC), and the amino acid sequences thereof were determined by nanoscale liquid chromatography-Orbitrap coupled to tandem mass spectrometry and *de novo* sequencing (nanoLC-Orbitap-MS/MS). In Example 2 the capacity of these peptides for inhibiting the activity of the angiotensin converting enzyme (ACE) is shown.

In view of the foregoing, the inventors have developed a set of inventive aspects which will be disclosed in detail below. The present invention is defined by the claims.

*Peptide of the invention*

The inventors have discovered that peptides comprising at least two cysteines separated by n amino acids, being n an integer of 0, 1, 2 or 3, and consisting of a length between 5 and 10 consecutive amino acids are capable of inhibiting the activity of the angiotensin converting enzyme (ACE), being thus useful as anti-hypertensive agents (see table 1 below).

**Table 1. Antihypertensive peptide sequences identified.**

| Sequence number | Peptide sequence | Number of amino acids | Molecular mass |
|---|---|---|---|
| SEQ ID N°1 | RDGGYCC | 7 | 772,86 |
| SEQ ID N°2 | YYCPADCPS | 9 | 1018,14 |
| SEQ ID N°3 | YGCGCDPL | 8 | 826,95 |
| SEQ ID N°4 | LEEFCC | 6 | 742,87 |
| SEQ ID N°5 | HCGCNTH | 7 | 770,85 |
| SEQ ID N°6 | WAAGYCC | 7 | 772,91 |
| SEQ ID N°7 | CCGNAVPQ | 8 | 790,92 |

The nomenclature used in the peptides listed in Table 1 is as follows: A, alanine; C, cysteine; D, aspartic acid; E, glutamic acid; F, phenylalanine; G, glycine; H, histidine; K, lysine; L, leucine; M, methionine; N asparagine; P, proline; Q, glutamine; R, arginine; S, serine; T, threonine; V, valine; W, tryptophan; Y, tyrosine. In the sequences, leucine (L) can be replaced by isoleucine (I).

Thus, in one aspect, the present invention relates to an isolated peptide, or any of salts, esters, solvates and anhydrates pharmaceutically acceptable thereof, hereinafter "peptide of the invention", comprising at least two cysteines separated by 0amino acids, consisting of a length between 8, 9 or 10 amino acids, wherein the peptide shows anti-hypertensive activity and comprises the sequence SEQ ID NO: 7.

The term "peptide" as used herein refers to a linear series of natural, non-natural and/or chemically modified amino acid residues connected one to the other by peptide bonds. The amino acid residues are represented throughout the specification and claims by either one or three-letter codes, as is commonly known in the art.

The term "isolated peptide" refers to a peptide that is essentially free from contaminating cellular components, such as carbohydrate, lipid or other proteinaceous impurities associated with the peptide in nature or that, if it is found in natural medium, has been synthetically (not naturally) altered by human intervention. Typically, a preparation of isolated peptide contains the peptide in a highly purified form, i.e., at least about 80% pure, at least about 90% pure, at least about 95% pure, greater than 95% pure, or greater than 99% pure.

In the context of the present invention, it is considered that a peptide shows "antihypertensive activity" when a peptide after being administered to a subject is capable of reducing or decreasing the blood pressure of said subject.

Essays for identifying compounds with antihypertensive activity are widely known in the state of the art. For example, inhibition of the angiotensive-converting enzyme (as shown in Example 2 which illustrate the present invention), or the administration of the compound to be tested to a model of spontaneously hypertensive rats and to analyze the changes in the systolic blood pressure and diastolic blood pressure (see Example 3).

The peptide of the invention can comprise any natural, non-natural and/or chemically modified amino acid residues in its structure. However, the inventors have additionally found that if the peptide comprises an aromatic amino acid in its structure, the peptide shows higher anti-hipertensive activity than those peptides which does not comprise aromatic amino acids. As the skilled person in the art knows, the aromatic amino acids are characterized by having in its structure an aromatic ring. Examples of aromatic acids includes, but not limited to, phenylalanine, tryptophan, histidine, tyrosine, thyroxine, 5-hydroxytryptophan and L-DOPA. In a particular embodiment, the aromatic amino acid is selected from the group consisting of phenylalanine, tyrosine and Tryptophan. Examples of peptides comprising aromatic amino acids includes, without limited to, peptides showing the sequences SEQ ID NO: 1, SEQ ID NO: 2, SEQ ID NO: 3, SEQ ID NO: 4 or SEQ ID NO:6.

As used herein, the terms "analog" and "derivative" are equivalents and refer to a peptide comprising at least one altered amino acid residue by an amino acid substitution, addition, deletion or chemical modification, as compared with the native peptide, but always comprising two cysteines separated by n amino acids, being n an integer of 0, 1, 2 or 3, consisting of a length between 5 and 10 amino acids, preferably, 6, 7, 8 or 9 amino acids, and showing anti-hypertensive activity. Peptide derivatives particularly include amino acid substitutions and/or additions with naturally occurring amino acid residues, and chemical modifications such as, for example, enzymatic modifications, typically present in nature. Peptide analogs particularly include amino acid substitutions and/or additions with non-natural amino acid residues, and chemical modifications which do not occur in nature. The analogs and derivatives comprise an amino acid sequence that is at least 60, 65, 70, 75, 80, 85, 90, 95, 96, 97, 98 or 99% identical to the amino acid sequence of SEQ ID NO: 1 to SEQ ID NO: 7.

In the present invention, the terms "identical", "sequence identity", "identity" and "similarity" are considered equivalents and can be used interchangeably. The term "sequence identity" (or its grammatical equivalents) means that a particular sequence has at least a certain percentage of amino acid residues identical to those in a specified reference sequence using an alignment algorithm. The degree of identity can be determined by, for example, the Clustal method, the Wilbur-Lipman method, the GAG program, including GAP, BLAST or BLASTN, EMBOSS Needle, FASTA, etc. Furthermore, the Smith Waterman algorithm can be used in order to determine the degree of identity between two sequences. An example of an algorithm that is suitable for determining sequence similarity is the BLAST algorithm, which is described in Altschul, et al, J. Mol. Biol. 215:403-410 (1990). Software for performing BLAST analyses is publicly available through the National Center for Biotechnology Information.

The peptide of the invention is a novel peptide which inhibit ACE and are absorbed intact from the digestive tract, preventing or reducing hypertension. The effective amount was determined for this peptide and was found to be between 0.05 mg and 10 mg per kg body weight per day, preferably between 0.1 mg and 1 mg per kg body weight per day, considering that the effective amount refers to the sum of the amounts of peptides included in this invention. This effective amount can reduce systolic blood pressure by 5 mm Hg, or more, in a pre-hypertensive or hypertensive subjects.

The peptides herein disclosed can be obtained from any source. The peptides may be obtained from the raw material coming from animal or vegetable fat. Examples of animal or vegetable fat include, but not limited to, olives, blubber of whales, sunflower seeds, olive oil, sunflower oil, soybean oil, rapeseed oil, corn oil, maize oil, palm oil, linseed oil, flaxseed oil, almond oil, avocado oil, walnut oil, coconut oil, castorbean oil, cottonseed oil, peanut oil, safflower oil, sesame oil, lard, butter, fat of pig, fat of goat, beef fat, cattle fat, buffalo fat, sheep fat, fat of poultry, fish oil, oil from algae and marine animal oils. The peptides may be obtained from raw material coming from mashing and/or milling of olives, such as, without limiting to, olive oil or by-products of the olive oil industry. Examples of by-products of the olive oil industry include, without limited to, crushed olives paste, pomace oil, olive pomace, olive mill wastewater, dried/defatted olive pomace cake and blends and mixtures thereof. In order to obtain the peptides disclosed herein, the raw materials are subjected to different processes of extraction with organic solvents which are widely known by the skilled person in the art.

The present invention also encompasses a fusion peptide comprising the peptide of the invention and a carrier or marker peptide. Thus, in another aspect, the present invention relates to a fusion peptide comprising the peptide of the invention and a carrier or marker peptide, wherein

- the carrier peptide is a peptide which internalizes a peptide in the cell, and
- the marker peptide is an amino acid sequence useful for the isolation or purification of the isolated peptide.

In the context of the present invention, a "carrier peptide" is defined as a peptide with capacity to internalize a peptide in the cell". The carrier peptide is a peptide capable of traversing the cell membrane and penetrating the cell from the outside, a characteristic which can be conferred to the peptide (e.g., peptide of the invention) to which it is fused (fusion protein of the invention), thus providing an alternative to the transport of peptides of interest (e.g., peptides of the invention) into the target cells. This mechanism of entrance of peptides into the cell is known as "protein transduction or delivery"). Various carrier peptides with capacity to internalize a peptide in a cell are known (Schwarze S.R. et al., Science, 1999 Sep 3; 285(5433):1569-72 ; Niesner U. et al., Bioconjug. Chem. 2002 Jul-Aug; 13(4):729-36 ; Ford K.G. et al., Gene Therapy, 2001; 8:1-4 ; and Gusarova G.A. et al., J. Clin. Invest. 2007 Jan; 117(1):99-111).

Virtually any carrier peptide with capacity to internalize a peptide in a cell can be used for putting the present invention into practice; nevertheless, said carrier peptide is a peptide comprising a "PTD" ("protein transduction domain") segment. Illustrative non-limiting examples of proteins comprising protein transduction domains (PTDs) include the human immunodeficiency virus 1 (HIV-1) TAT ("transacting translational protein") protein, the Drosophila antennapedia homeotic transcription factor (Antp) and the herpesvirus simplex 1 (HSV-1) VP22 DNA-binding protein, although it has also been suggested that other proteins have this property of internalizing peptides in cells, such as influenza virus hemagglutinin, lactoferrin, fibroblast growth factor-1, fibroblast growth factor-2 and the Hoxa-5, Hoxb-4 and Hoxc-8 proteins (Ford K.G. et al., Gene Therapy, 2001; 8:1-4). Examples of carrier peptide include, without limiting to,

- a peptide derived from the HIV-1 TAT protein, comprising the sequence responsible for peptide transduction, the basic domain (PTD) of which comprises moieties 49-57 of said HIV-1 TAT protein, specifically the amino acid sequence RKKRRQRRR (SEQ ID NO: 8), or moieties 47-57 of said HIV-1 TAT protein, such as the peptide the amino acid sequence of which is YGRKKRRQRRR (SEQ ID NO: 9) or the peptide the amino acid sequence of which is CGISYGRKKRRQRRR (SEQ ID NO: 10);

- a peptide derived from the D. antennapedia Antp protein, comprising the antennapedia homeodomain (AntpHD) comprising the domain responsible for peptide transduction (PTD) [moieties 43-58 of said Antp protein), comprising the amino acid sequence RQIKIWFQNRRMKWKK (SEQ ID NO: 11), or a functional fragment thereof;

- a peptide derived from the HSV-1 VP22 protein comprising a domain responsible for peptide transduction (PTD); and

- a peptide derived from the ARF ("alternative reading frame") tumor suppressing protein comprising the amino acid sequence responsible for the capacity of the peptide of penetrating the cells, such as the fragment comprising moieties 26-44 of said ARF protein, specifically, the amino acid sequence KFVRSRRPRTASCALAFVN (SEQ ID NO: 12), or a fragment thereof comprising moieties 37-44 of said ARF protein, specifically the amino acid sequence SCALAFVN (SEQ ID NO: 13).

The peptide of the invention can be bound to any of the (amino or carboxyl) terminal ends of the carrier peptide with capacity to internalize a peptide of the invention in a cell, and may or may not be directly bound to said carrier peptide with capacity to internalize a peptide in a cell. Therefore, the peptide of the invention is directly bound to said carrier peptide or, alternatively, is bound to through a linker or spacer peptide between both peptides. Any peptide with structural flexibility can be used as a spacer peptide; nevertheless, illustrative non-limiting examples of said spacer peptides include peptides containing repeats of amino acid moieties, e.g., of Gly and/or Ser, or any other suitable repeat of amino acid moieties.

In the context of the present invention, a marker peptide is an amino acid sequence useful for the isolation or purification of the fusion protein of the invention. Said sequence will be located in a region of the fusion protein of the invention which does not adversely affect the functionality of the peptide of the invention. Virtually any amino acid sequence which can be used to isolate or purify a fusion protein (generically called tag peptides) can be present in said fusion protein of the invention. By way of a non-limiting illustration, said amino acid sequence useful for isolating or purifying a fusion protein can be, for example, an arginine tag (Arg-tag), a histidine tag (His-tag), FLAG-tag, Strep-tag, an epitope which can be recognized by an antibody, such as c-myc-tag, SBP-tag, S-tag, calmodulin-binding peptide, cellulose-binding domain, chitin-binding domain, glutathione S-transferase-tag, maltose-binding protein, NusA, TrxA, DsbA, Avi-tag, etc. (Terpe K., Appl. Microbiol. Biotechnol. (2003), 60:523-525), β-galactosidase, VSV-glycoprotein (YTDIEMNRLGK) (SEQ ID NO: 14), or an amino acid sequence such as: AHGHRP (SEQ ID NO: 15) (2, 4, and 8 copies), PIHDHDHPHLVIHS (SEQ ID NO: 16), etc.

Additionally, the peptides disclosed in the present disclosure may be part of a composition together with other compounds useful for its administration to a subject. Thus, the present disclosure relates to a composition comprising the peptides disclosed herein or a derivative thereof.

The term "composition" refers to any combination of one or more substances wherein at least one of said substances is the peptides disclosed herein. Compositions may be a variety of kinds, including, but not limited to, peptide extracts, nutritional supplements, pharmaceutical preparations, vitamin supplements, food additives or foods supplements. The terms "pharmaceutical compositions" and "formulations" are used interchangeably herein.

The composition disclosed herein is a peptide extract, a nutritional composition, a food, a supplement, a nutraceutical, a probiotic or a symbiotic.

The term "peptide extract" refers to a preparation containing the active ingredient of a substance in concentrated form. In the context of the present invention, the active ingredient is the peptides of of the present disclosure.

The term "nutritional composition" relates to a food that regardless of providing nutrients to the subject eating them has a beneficial effect on one or more bodily functions, so as to provide a better state of health and well-being. Accordingly, such nutritional composition may be destined for the prevention and/or treatment of a disease or for the reduction of disease risk factors.

The term "supplement", which is synonymous with any of the terms "dietary supplement", "nutritional supplement" or "food supplement", refers to a component or components destined for supplementing the diet and may be a food. Examples of dietary supplements are, but not limited to, vitamins, minerals, botanical products, amino acids and food components such as enzymes and glandular extracts. They are not presented as a substitute for a conventional food or as the sole component of a meal or diet, but rather as a dietary supplement.

The term "nutraceutical" as used herein relates to the isolated substances of a food used in dosage form and having a beneficial effect on human health. Said nutraceutical can be a supplement.

The term "symbiotic" as used herein relates to those foods which contain a mixture of prebiotics and probiotics. As a general rule, they contain a prebiotic component to enhance the growth and/or metabolic activity and, ultimately, the effect of the probiotic with which it is combined, such as for example, but not limited to, the association of fructooligosaccharides and galactooligosaccharides.

The composition of the present disclosure may be a food selected from the group consisting of a beverage, infused food, milk, yogurt, cheese, flavoured milk drink, bread, cake, butter, margarine, a sauce, a condiment, a salad dressing, mayonnaise, fruit juice, syrup, a dessert, icings and fillings, a soft frozen product, a confection, a chewing gum and an intermediate food.

In addition, the composition comprises a prophylactically or therapeutically effective amount of the peptides disclosed herein or a derivative thereof.

The term "prophylactically effective amount" as used herein means that the peptides contained in the composition is in sufficient quantity to achieve the intended purpose, such as, in this case, to prevent or avoid an increase of the blood pressure of a subject. The term "therapeutically effective amount" as used herein means that the peptides contained in the composition is in sufficient quantity to achieve the intended purpose, such as, in this case, to reduce or decrease the blood pressure of a subject. The effective amount is between 0.05 mg and 10 mg per Kg body weight per day, preferably between 0.1 mg and 1 mg per Kg body weight per day. In case that the composition comprises a combination of peptides as explained below, said effective amount refers to the sum of the amount of the peptides. Thus effective amount can reduced systolic blood pressure by 5 mmHg, or more, in a pre-hypertensive or hypertensive subjects.

A decrease in the blood pressure may be measured by the decrease of the systolic blood pressure and/or diastolic blood pressure. Methods of measuring the blood pressure are well known in the art, and need not be repeated herein. For example, the blood pressure may be measure by using auscultatoric measurement devices, such as stethoscopes or sphygmomanometer, or by oscillometric measurement devices which use an electronic pressure sensor with a numerical readout of blood pressure.

The composition disclosed herein may comprise the peptide SEQ ID NO: 1, SEQ ID NO: 2, SEQ ID NO: 3, SEQ ID NO: 4, SEQ ID NO: 5, SEQ ID NO: 6and/or combinations thereof. In a more particular embodiment, the composition comprises the following combinations of peptides:

- SEQ ID NO: 1 and SEQ ID NO: 2;
- SEQ ID NO: 1 and SEQ ID NO: 3;
- SEQ ID NO: 1 and SEQ ID NO: 4;
- SEQ ID NO: 1 and SEQ ID NO: 5;
- SEQ ID NO: 1 and SEQ ID NO: 6;
- SEQ ID NO: 1, SEQ ID NO: 2 and SEQ ID NO: 3;
- SEQ ID NO: 1, SEQ ID NO: 2 and SEQ ID NO: 4;
- SEQ ID NO: 1, SEQ ID NO: 2 and SEQ ID NO: 5;
- SEQ ID NO: 1, SEQ ID NO: 2 and SEQ ID NO: 6;
- SEQ ID NO: 1, SEQ ID NO: 2, SEQ ID NO: 3 and SEQ ID NO: 4;
- SEQ ID NO: 1, SEQ ID NO: 2, SEQ ID NO: 3 and SEQ ID NO: 5;
- SEQ ID NO: 1, SEQ ID NO: 2, SEQ ID NO: 3 and SEQ ID NO: 6;
- SEQ ID NO: 1, SEQ ID NO: 2, SEQ ID NO: 3 and SEQ ID NO: 4 and SEQ ID NO: 5;
- SEQ ID NO: 1, SEQ ID NO: 2, SEQ ID NO: 3 and SEQ ID NO: 4 and SEQ ID NO: 6;
- SEQ ID NO: 1, SEQ ID NO: 2, SEQ ID NO: 3 and SEQ ID NO: 4, SEQ ID NO: 5 and SEQ ID NO: 6;
- SEQ ID NO: 2 and SEQ ID NO: 3;
- SEQ ID NO: 2 and SEQ ID NO: 4;
- SEQ ID NO: 2 and SEQ ID NO: 5;
- SEQ ID NO: 2 and SEQ ID NO: 6;
- SEQ ID NO: 2, SEQ ID NO: 3 and SEQ ID NO: 4;

- SEQ ID NO: 2, SEQ ID NO: 3 and SEQ ID NO: 5;
- SEQ ID NO: 2, SEQ ID NO: 3 and SEQ ID NO: 6;
- SEQ ID NO: 2, SEQ ID NO: 3, SEQ ID NO: 4 and SEQ ID NO: 5;
- SEQ ID NO: 2, SEQ ID NO: 3, SEQ ID NO: 4 and SEQ ID NO: 6;
- SEQ ID NO: 2, SEQ ID NO: 3, SEQ ID NO: 4, SEQ ID NO: 5 and SEQ ID NO: 6;
- SEQ ID NO: 3 and SEQ ID NO: 4;
- SEQ ID NO: 3 and SEQ ID NO: 5;
- SEQ ID NO: 3 and SEQ ID NO: 6;
- SEQ ID NO: 3, SEQ ID NO: 4 and SEQ ID NO: 5;
- SEQ ID NO: 3, SEQ ID NO: 4 and SEQ ID NO: 6;
- SEQ ID NO: 3, SEQ ID NO: 4, SEQ ID NO: 5 and SEQ ID NO: 6;
- SEQ ID NO: 4 and SEQ ID NO: 5;
- SEQ ID NO: 4 and SEQ ID NO: 6;
- SEQ ID NO: 4, SEQ ID NO: 5 and SEQ ID NO: 6;
- SEQ ID NO: 5 and SEQ ID NO: 6;

Compositions may include a carrier. Depending on the kind of compositions, a carrier may be a dietary suitable carrier or a pharmaceutically acceptable carrier, as long as it is compatible with the particular kind of composition. Examples of a dietary suitable carrier include, but are not limited to, dietary suitable excipients, diluents, and carriers.

The term "excipient" relates to a substance that aids the absorption of any of the components of the composition of the present invention, stabilises said components or aids the preparation of the composition in the sense of giving consistency or contributing flavours that make it more enjoyable. Thus, excipients may have the function of holding the components together, such as starches, sugars or celluloses, a sweetening function, colouring function, drug protection function such as to isolate from the air and/or humidity, the function of filling a tablet, capsule or other form of presentation such as, for example, dibasic calcium phosphate, a disintegrating function to facilitate the dissolution of the components and their absorption in the intestine, without excluding any other type of excipients not mentioned in this paragraph. Therefore, the term "excipient" is defined as the material included in the galenic forms, is added to the active ingredients or their associations to enable their preparation and stability, modify their organoleptic properties or determine the physico-chemical properties of the pharmaceutical composition and its bioavailability.

Examples of a pharmaceutically acceptable carrier include, but are not limited to, biocompatible vehicles, adjuvants, additives, and diluents to achieve a composition usable as a dosage form. As used herein, the terms "pharmaceutically acceptable," "physiologically tolerable," and grammatical variations thereof, as they refer to compositions, carriers, diluents, and reagents, are used interchangeably and represent that the materials are capable of administration to or upon a mammal without the production of undesirable physiological effects.

Likewise, the compositions disclosed herein may be used alone or in combination with other biologically active ingredients. Examples of biologically active ingredients include, without limited to, vitamins, minerals, botanical products, amino acids, enzymes, glandular extracts, probiotics, oligosaccharides, etc. The term "probiotic" as used herein relates to microorganisms which, when administered in adequate amounts, have beneficial effects on the health of the host organism.

A composition, alone or in combination with other active ingredients, may be administered to a subject in a single dose or multiple doses over a period of time, generally by oral administration. Various administration patterns will be apparent to those skilled in the art. The dosage ranges for the administration of the compositions are those large enough to produce the desired effect. The dosage should not be so large as to cause any adverse side effects, such as unwanted cross-reactions and the like. Generally, the dosage will vary with the age, weight, sex, condition, and extent of a condition in a subject, and the intended purpose. The dosage can be determined by one of skill in the art without undue experimentation. The dosage can be adjusted in the event of any counter indications, tolerance, or similar conditions. Those of skill in the art can readily evaluate such factors and, based on this information, determine the particular effective concentration of a composition to be used for an intended purpose. For example, the concentration of the peptides in a single-dose formulation is from 0.0005% to 95% of the total formulation.

The term "dosage unit" as used herein refers to physically discrete units suitable as unitary dosages for animals, each unit containing a predetermined quantity of active material calculated to produce the desired therapeutic effect in association with the required diluent, e.g., a carrier or vehicle. The specifications for the unit dose are dictated by and are directly dependent on (a) the unique characteristics of the active material and (b) the limitations inherent in the art of compounding such active material for therapeutical use in animals.

In each case the galenic form of the pharmaceutical composition and, therefore, the drug, will be adapted to the dosage form used. Therefore, the composition disclosed herein can be provided in the form of solutions or any other clinically permitted dosage form and in a therapeutically effective quantity. The pharmaceutical composition may be formulated in solid, semisolid, liquid or gaseous forms, such as tablet, capsule, powder, granule, ointment, solution, suppository, injection, inhalant, gel, microsphere or aerosol. The form adapted for oral administration relates to a physical state which would permit its oral administration. Said form adapted for oral administration is selected from the list comprising, but not limited to, drops, syrup, herbal tea, elixir, suspension, extemporaneous suspension, drinkable phial, tablet, capsule, granule, wafer, pill, tablet, lozenge, troche or lyophilised. Alternatively, the pharmaceutical composition may also be presented in a form adapted for sublingual, nasal, intrathecal, bronchial, lymphatic, rectal, transdermal, inhaled or parenteral administration.

*Uses of the peptide of the invention*

As explained previously, the inventors of the present invention has discovered that the peptide of the invention is, surprisingly, capable of inhibiting the activity of the angiotensin converting enzyme (ACE), being thus useful as anti-hypertensive agents.

Therefore, in another aspect, the invention relates to the peptide of the invention for use as a medicament.

In another aspect, the invention relates to the peptide of the invention for use in the treatment and/or prevention of pre-hypertension or hypertension.

As used herein, "treatment," "treat," or "treating," refers to: (a) preventing the disease or condition from occurring in a subject which may be predisposed to the disease or condition but has not yet been diagnosed as having it; (b) inhibiting the disease or condition, i.e., arresting its development; (c) relieving and or ameliorating the disease or condition, i.e., causing regression of the disease or condition; or (d) curing the disease or condition, i.e., stopping its development or progression. The population of subjects treated by the stem cell, the cell population, the conditioned medium or the pharmaceutical composition of the invention includes subjects suffering from the undesirable condition or disease, as well as subjects at risk for development of the condition or disease. In the present invention, the diseases to be treated are selected from pre-hypertension and hypertension.

The terms "disorder" and '"disease" are used interchangeably to refer to a condition in a subject. The term "subject" in the above-mentioned definitions refers to any animal, including, but not limited to, mammals, preferably primates, more preferably humans. Thus, the peptide or the composition of the invention can be used in the treatment of any animal suffering from the above-mentioned diseases.

In the context of the present invention, the term "pre-hypertension" relates to a Slightly elevated blood pressure, having a systolic pressure from 120 to 139 millimeters of mercury (mm Hg) or a diastolic pressure from 80 to 89 mm Hg. Prehypertension will likely turn into high blood pressure (hypertension) unless you make lifestyle changes, such as getting more exercise and eating healthier foods. Both prehypertension and high blood pressure increase your risk of heart attack, stroke and heart failure. The term "hypertension" as used herein refers to a systolic arterial blood pressure of 140 mm Hg or higher and a diastolic arterial blood pressure of 90 mm Hg or higher. Methods for measure the blood pressure have been cited in previous paragraphs.

The term "stroke", also known as cerebrovascular accident (CVA), cerebrovascular insult (CVI), or brain attack, is when poor blood flow to the brain results in cell death. There are two main types of stroke: ischemic, due to lack of blood flow, and hemorrhagic, due to bleeding. The main risk factor for stroke is high blood pressure.

The term "coronary disease" or "coronary heart disease" or CHD, also known as ischemic heart disease (IHD), atherosclerotic heart disease, atherosclerotic cardiovascular disease and coronary heart disease, refers to a disease in which a waxy substance called plaque builds up inside the coronary arteries which supply oxygen-rich blood to your heart muscle. When plaque builds up in the arteries, the condition is called atherosclerosis.

The term "myocardial infarction" (MI) or acute myocardial infarction (AMI), commonly known as a heart attack, refers to a condition in which the blood flow stops to a part of the heart causing damage to the heart muscle.

The term "metabolic syndrome" as used herein refers to a cluster of conditions - increased blood pressure, a high blood sugar level, excess body fat around the waist and abnormal cholesterol levels - that occur together, increasing the risk of heart disease, stroke and diabetes.

The term "peripheral artery disease" (also called peripheral arterial disease) as used herein refers to a common circulatory problem in which narrowed arteries reduce blood flow to your limbs.

The term "an abdominal aortic aneurysm", also known as a triple-a, as used herein refers to a localized enlargement of the abdominal aorta such that the diameter is greater than 3 cm or more than 50% larger than normal. They usually cause no symptoms except when ruptured. Occasionally there may be abdominal, back or leg pain. Large aneurysms can sometimes be felt by pushing on the abdomen. Rupture may result in pain in the abdomen or back, low blood pressure or a brief loss of consciousness.

As the skilled person in the art understands, the peptides or composition disclosed herein may be administered in combination with other drugs to stop or slow some of the body's functions that cause high blood pressure. Examples of drug to lower blood pressure include, but not limited to,

- Diuretics (Water or Fluid Pills): Flush excess sodium from the body, which reduces the amount of fluid in the blood and helps to lower the blood pressure.

- Beta Blockers: Help the heart beat slower and with less force. As a result, the heart pumps less blood through the blood vessels, which can help to lower the blood pressure.
- Angiotensin-Converting Enzyme (ACE) Inhibitors: Angiotensin-II is a hormone that narrows blood vessels, increasing blood pressure. ACE converts Angiotensin I to Angiotensin II. ACE inhibitors block this process, which stops the production of Angiotensin II, lowering blood pressure.
- Angiotensin II Receptor Blockers (ARBs): Block angiotensin II hormone from binding with receptors in the blood vessels. When angiotensin II is blocked, the blood vessels do not constrict or narrow, which can lower the blood pressure.
- Calcium Channel Blockers: Keep calcium from entering the muscle cells of the heart and blood vessels. This allows blood vessels to relax, which can lower the blood pressure.
- Alpha Blockers: Reduce nerve impulses that tighten blood vessels. This allows blood to flow more freely, causing blood pressure to go down.
- Alpha-Beta Blockers: Reduce nerve impulses the same way alpha blockers do. However, like beta blockers, they also slow the heartbeat. As a result, blood pressure goes down.
- Central Acting Agents: Act in the brain to decrease nerve signals that narrow blood vessels, which can lower blood pressure.
- Vasodilators: Relax the muscles in blood vessel walls, which can lower blood pressure.

Alternatively, the peptides and compositions disclosed herein can be used for elaborating food with antihypertensive properties, commonly called functional foods. By way of non-limiting illustration, examples or functional food include beverage, infused food, milk, yogurt, cheese, flavoured milk drink, bread, cake, butter, margarine, a sauce, a condiment, a salad dressing, mayonnaise, fruit juice, syrup, a dessert, icings and fillings, a soft frozen product, a confection, a chewing gum, bread, etc.

*Method for obtaining the peptide of the invention*

In another aspect, the present invention relates to a method for obtaining the peptide of the invention, hereinafter "method of the invention", comprising

(i) obtaining a protein precipitate from oil coming from olives,
(ii) obtaining a peptide extract from the precipitate of step (i) comprising the peptide of the invention, and
(iii) Fractioning and purifying the peptide of the invention.

the peptides disclosed herein can be obtained from animal or vegetable fat. Any animal or vegetable fat can be used for obtaining the oil from which the peptide of the invention is going be isolated. Examples of oil coming from animal or vegetable fat include, but without limiting to, olive oil, sunflower oil, soybean oil, rapeseed oil, corn oil, maize oil, palm oil, linseed oil, flaxseed oil, almond oil, avocado oil, walnut oil, coconut oil, castorbean oil, cottonseed oil, peanut oil, safflower oil, sesame oil, lard, butter, fat of pig, fat of goat, beef fat, cattle fat, buffalo fat, sheep fat, fat of poultry, fish oil, oil from algae, marine oils

Methods for processing the different sources of animal or vegetable fat for obtaining oil are widely known in the state of the art. Examples of these processes are, without limiting to, mashing and/or milling by physical procedures. The mashing and/or milling of the sources of animal or vegetable fat results in oil and other products (also called derived products or by-products) which, as the skilled person in the art understands, may be processed again for obtaining more oil.

In the present invention, in order to obtained oil coming from olives as defined in step (i), the olives are mashed and/or milled resulting in oil and by-products such as pomade, crushed olives paste, olive mill wastewater, etc., which will be used for obtaining the peptide of the invention. Thus, in the present invention, the oil from which the peptide of the invention is going to be isolated may come from the oil directly obtained from the processing of olives or from by-products derived from the physical treatment thereof. Example of by-products derived from the processing of the animal or vegetable fat are, without limited to crushed olives paste, pomace oil, olive pomace, olive mill wastewater, dried/defatted olive pomace cake, and blends. Thus, in a particular embodiment, the oil used in the method of the invention comes from mashing and/or milling of olives, in another particular embodiment, the oil comes from the group consisting of crushed olives paste, pomace oil, olive pomace, olive mill wastewater, dried/defatted olive pomace cake, and blends and mixtures thereof.

As the skilled person in the art understands, in order to obtain oil from the by-products derived from the processing of olives, they have to be treated. Examples of the different proceedings which can be used for this purpose include, without limiting to:

- Crushed olive paste

The crushed olive paste obtained from the milling of the olives contains olive oil and it is a source of antihypertensive peptides. The procedure to obtain the peptides is to remove first the residual water moisture of the starting material using the standard procedures available. Then, the oil is extracted with hexane. The products resulting from the extraction are hexane extract of olive oil and defatted olive pulp. The hexane is evaporated and the olive oil is obtained.

- Olive pomace.

Olive pomace is the solid remains of olives after the olive oil extraction. To obtain the peptides from olive pomace, the first step is to remove the residual water moisture of the pomace using the standard procedures available. Next, the remaining oil is extracted using hexane as solvent. Two fractions are obtained: hexane-extracted oil and a defatted pomace residue (or cake). Evaporation of the hexane originates the extracted oil, namely pomace oil.

- Olive mill wastewater.

Olive mill wastewater contains a high percentage of water which has to be removed first by using conventional drying methods. Then, extraction of the residual oil (0.5-1%) is performed using hexane as a solvent, which is subsequently evaporated.

- Olive pomace cake.

The olive pomace is first dried to remove the residual moisture using standard procedures. The oil present in the desiccated pomace is then extracted using hexane as solvent, followed by evaporation of the hexane. The dried and defatted pomace cake obtained is then subjected to a process of solubilisation of proteins and peptides at pH 10.5.

The peptides disclosed herein may also be obtained from any starting material of plant or animal origin containing the amino acid sequences of bioactive peptides of interest, by a process for hydrolysis of raw materials using proteolytic enzymes. This starting or raw material is dissolved or dispersed, at a suitable concentration, in water or in buffered solution. The pH of the solution has to be adequate for the activity of a protease which is capable of degrading the proteins of the starting material, producing the peptides of interest. Proteases can be any endo-peptidase or exo-peptidase such as trypsin, chymotrypsin, elastase, thrombin, subtilisin, alcalase, flavourzyme, pepsin, pancreatin etc. More specifically, alcalase and flavourzyme can be used at pH 8 and pH 7, respectively. The hydrolysis is carried out at 37°C for a period of between 10 minutes and 12 hours, preferably 45 minutes. The enzymes are inactivated by heating at 95°C.

The method of the invention may comprise obtaining a protein precipitate from oil. Any method for obtaining a protein precipitate from oil can be used in the context of the present invention. For example, a volume of a mixture of organic solvents, preferably acetone/hexane (1:1), may be added to the olive oil obtained from the previous step. Then, the mixture may be centrifuged at 10,000g for 15 minutes at 4ºC and the pellets (or protein precipitate), containing the peptides, are collected. This step may be performed between 2 and 7 times, preferably between 3 and 5 times.

Step (ii) of the method of the invention comprises obtaining a peptide extract from the precipitate of step (i) comprising the peptide of the invention.

Methods for obtaining a peptide extract from the precipitate are widely known from the state of the art. The collected pellets may be, for example, brought to dryness at a temperature below 40°C, obtaining a precipitate which is resuspended, and the olive oil peptides may be extracted using appropriate mixtures of water and acetonitrile, preferably 80:20, at 4°C. Then, the extract may undergo a process of sonication for above 30 minutes and centrifuged again. The supernatants containing the concentrated olive oil peptides will be collected.

Finally, the method of the invention comprises a step (iii) comprising the fractionation and purification of the peptide of the invention.

The term "purify" as used in this description refers to the isolation of the peptide of the invention and to its concentration from the other peptides present in the culture medium. Isolation can be carried out by means of differential solubility techniques, chromatography, electrophoresis or isoelectric focusing. Chromatographic techniques can be based on the molecular weight, ionic charge (based on the ionisation state of the amino acids in working conditions), affinity of the protein for certain chromatographic matrices or columns or by purification tags and can be performed in columns, on paper or plates. Isolation of the peptide can be performed, for example, by precipitation with ammonium sulphate, fast protein liquid chromatography (FPLC) or high performance liquid chromatography (HPLC), using automated systems that notably reduce purification time and increase purification yield.

In the present invention, the concentrated olive oil peptides obtained in the previous step can be fractionated by size-exclusion chromatography, using a fast protein liquid chromatography system (FPLC) and a molecular exclusion column specific for peptides. For this purpose, a certain amount of concentrate peptides from the previous step is injected onto the FPLC system. The separation of the peptides is carried out using a mixture of water/acetonitrile (80:20) with 0.1% trifluoroacetic acid. As the skilled person in the art knows, reverse phase high-performance liquid chromatography (RP-HPLC) can be used as well. As can be seen from the examples of the present invention, by putting into practice this step, can be obtained several fractions comprising purified peptides (see Table 3).

Alternatively to, or following step (iii), the peptide of the invention can be concentrated by ultrafiltration, dialysis, electro dialysis and can be dried by lyophilisation, atomization, evaporation, etc., producing a dried concentrate containing the peptide of the invention.

As indicated above, the peptides disclosed herein can be obtained by the hydrolysis of animal or vegetable fat using proteolytic enzymes. This starting material (sources of animal or vegetable fat) is dissolved or dispersed, at a suitable concentration, in water or in buffered solution. The pH of the solution has to be adequate for the activity of a protease which is capable of degrading the proteins of the starting material, producing the peptides of interest. Proteases can be any endo-peptidase or exo-peptidase such as trypsin, chymotrypsin, elastase, thrombin, subtilisin, alcalase, flavourzyme, pepsin, pancreatin etc. More specifically, alcalase and flavourzyme can be used at pH 8 and pH 7, respectively. The hydrolysis is carried out at 37°C for a period of between 10 minutes and 12 hours, preferably 45 minutes. The enzymes are inactivated by heating at 95°C. Thus, in a particular embodiment, the step (i) of the method of the invention comprises the hydrolysis of the raw material by a protease, preferably, the protease is alcalase or flavourzyme. In addition, microorganisms or bacterial fractions that can produce protein hydrolysis which originate peptides can also be used. The hydrolysate solutions obtained with these methodologies are desiccated using conventional methods.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings and sequence listing are provided by way of illustration and are not intended to be limiting of the present invention.

## BRIEF DESCRIPTION OF THE DRAWINGS

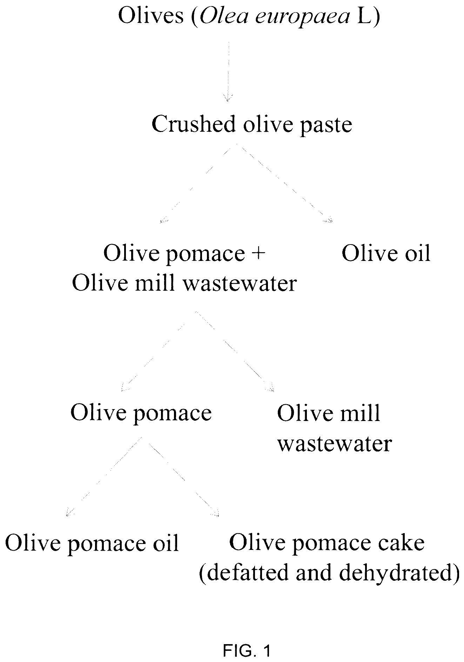

Figure 1. Preferred sources of antihypertensive peptides of the invention.

Figure 2. Molecular weight distribution of the concentrated peptides extracted from olive obtained by FPLC

Figure 3. Analysis by nano-Orbitrap LC MS/MS of extracted peptides from olive oil. The total ion chromatogram of the concentrated peptides is shown. Retention times of the most relevant peptides are shown. The table shows the peptide sequences and retention times identified in the chromatogram.

Figure 4. Panel A: angiotensin-converting enzyme inhibitory activity (ACEi) of concentrated peptides extracted from olive oil (1/2 to 1/128 dilutions). Panel B: calibration curve obtained from the data of panel A and used to determine $IC_{50}$.

Figure 5. Systolic blood pressure (SBP, Panel A) and diastolic blood pressure (DBP, Panel B) variations detected in spontaneously hypertensive rats (SHR) produced by the administration of: water (control group, O); Captopril 50 mg/Kg (□); olive oil concentrated peptides (10 mg/Kg) (■); olive oil concentrated peptides (100 mg/Kg) (▲); olive oil concentrated peptide (250 mg/Kg) (♦). The data shown are mean values $\pm$ standard error of the means. *, P <0.05 compared to the control group.

## EXAMPLES

## MATERIAL AND METHODS

Determination of angiotensin-converting enzyme inhibitory activity (ACEi)

The ACE inhibitory activity was determined according to the method described in Sentandreu and Toldrá (2006) [Sentandreu, M.A., Toldrá, F. A fluorescence-based protocol for quantifying angiotensin-converting enzyme activity. 2006. Nat. Protoc., 1 (5), 2423-2427], with some modifications. This assay is based on the ability of ACE to hydrolyse the substrate o-aminobenzoylglycyl-p-nitrophenylalanyl-proline (Abz-Gly-Phe-(NO$_2$)-Pro, Bachem Feinchemikalien, Suiza), producing the fluorescent product o-aminobenzoylglycine (Abz-Gly). The following reagents were used: buffer A: 150 mM Tris-HCl buffer (pH 8.3), with 0.1 $\mu$M ZnCl2; buffer B: 150 mM de Tris-HCl buffer (pH 8.4), with 1.125 M NaCl; ACE solution: rabbit-lung ACE (Sigma), previously dissolved in 50% glycerol, was diluted in buffer A to make an enzyme concentration of 0.042 U/mL. This solution was prepared fresh every day to conduct the experiment. Substrate solution: Abz-Gly-Phe(NO$_2$)-Pro was dissolved in buffer B to a final concentration of 0.45 mM at pH 8.3. This solution was also prepared every day before its use and was protected from light and kept at 4ºC.

The assay was carried out using a fluorescence technique. Black polystyrene plates of 96 wells (Thermo Scientific, USA) were used. The wells contained the following solutions: control: 40 μL of water and 40 μL of ACE solution; blank: 40 μL of water and 40 μL of buffer A; sample: 40 μL of inhibitor sample and 40 μL of ACE solution; sample blank: 40 μL of inhibitor sample and 40 μL of buffer A. The enzymatic reaction was initiated by adding 160 μL (final volume in each well 240 μL) of substrate solution and, immediately, the plate was mixed and incubated at 37°C in a VICTOR X5 fluorometer (PerkinElmer, USA). The fluorescence generated is measured after 30 minutes using 355 and 420 nm as excitation and emission wavelengths, respectively. The ACE inhibitory activity of each sample was determined in triplicate.

The ACE inhibitory activity was calculated using the following formula:

$$\text{ACE inhibitory activity } (\%) = \frac{(F_C - F_B) - (F_M - F_{Bm})}{F_C - F_B} \times 100$$

$F_C$ (Control): Fluorescence emitted after the action of ACE on the substrate, without inhibitor (i.e. sample).

$F_M$ (Sample): Fluorescence emitted after the action of ACE on the substrate, with inhibitor sample.

$F_B$: (Blank): Fluorescence emitted by the substrate.

$F_{Bm}$ (Sample Blank): Fluorescence emitted by the substrate and the sample.

The ACE inhibitory activity is expressed as IC$_{50}$ which is the concentration of inhibitor required to inhibit the activity of ACE by 50%.

Isolation of peptide fractions by size-exclusion chromatography using fast protein liquid chromatography (FPLC)

The peptide concentrates of the invention, obtained by the methods described above, was fractionated by gel filtration chromatography using a purification system of peptides and proteins "ÄKTApurifier" (GE Healthcare, England) equipped with a Superdex Peptide 10/300 GL (GE Healthcare) size-exclusion column with a separation range of between 0.1 and 7.0 kDa. The elution of the samples was conducted using at an isocratic method with 20% acetonitrile with 0.1% trifluoroacetic acid and flow of 0.8 mL/minute for 64 minute. The elution was monitored at 280 nm. Standard proteins of known molecular mass were used for the calibration of the size exclusion chromatographic column. The proteins used were cytochrome C (12,384 Da), aprotinin (6,512 Da), Vitamin B12 (1,355 Da) and tryptophan (204 Da) (Sigma-Aldrich, St. Louis, MO, USA). The injection volume of the samples and standards into the ÄKTApurifier was 200 μL. Seventeen 2-mL fractions were collected after each injection. Fractions were pooled into 6 groups (F1-F6) based on the chromatographic profile recorded at 280 nm of absorbance. Each of the fractions F1-F6 were dried using a Buchi rotary evaporator R-205 (BUCHI Labortechnik AG, Switzerland).

Peptide concentration measurements

The peptide concentration was determined using a protein quantification kit (FluoroProfile, Sigma-Aldrich). Bovine serum albumin was used as standard. Fluorimetric quantification was carried out to 530 nm and 630 nm as excitation and emission wavelengths, respectively.

Identification of peptides by nanoLC-Orbitrap-MS/MS and *de novo* sequencing

The sequence of peptides in olive oil and in by-products of the olive oil industry was determined by nanoscale liquid chromatography-Orbitrap coupled to tandem mass spectrometry and *de novo* sequencing (nanoLC-Orbitap-MS/MS). The following steps were used.

a) Sample preparation. The samples were diluted with milli Q water and 0.1% formic acid to a final protein concentration of 0.25 mg/mL.

b) Nano liquid chromatography and mass spectrometry analysis. Approximately 1 μg of total protein was injected in the system and analysed in triplicates. The peptides were separated onto a C-18 reversed phase nano-column (75μm id x 15cm; 3 μm, Nikkyo Technos Co., Japan) coupled to a nano-precolumn (100 μm id x 2 cm, 5 μm, Thermo Fisher Scientific, USA). The chromatographic separation was performed with 0.1% formic acid in water (phase A) and 0.1% formic acid in acetonitrile (phase B), using the following gradient: 0 to 5% B in 4 min, 5 to 15% of B in 60 min, 15 to 35% B in 60 min and 35 to 95% B in 10 min, maintained for 20 minutes. A flow rate of 300 nl/minutes was used to elute peptides for real time ionisation and peptide fragmentation on an LTQ-Orbitrap Veilos Pro mass spectrometer (Thermo Fisher). An enhanced FT-resolution spectrum (resolution = 30,000 FHMW) followed by a data dependent MS/MS scan. The data dependent MS/MS event consists on CID fragmentation (35% normalized collision energy) and IT-MS/MS acquisition from the most intense ten parent ions with a charge state rejection of one and dynamic exclusion of 0.5 minutes which is used for peptide identification

c) Peptide identification by *de novo* sequencing. *De novo* sequencing was performed using Peaks Studio 7 software. [Sin Ma, Kaizhong Zhang, Christopher Hendrie, Chengzhi Liang, Ming Li, Amanda Doherty-Kirby, Gilles Lajoie. PEAKS: Powerful Software for Peptide De Novo Sequencing by MS/MS. Rapid Communications in Mass Spectrometry, 17(20):2337-2342.2003]. The software extracts and deconvolutes MS and MS/MS spectra from samples analysed, comparing the obtain product ions scans with the theoretical mass fragments expected for those peptides that match with the exact mass measured in MS and MS/MS scans. Peaks software assigns a local confidence score for each amino acid in de novo sequences. The local confidence score ranges from 0% to 99%, indicating how confident the algorithm considers a particular amino acid is correctly sequenced. Moreover, the peptide sequence is evaluated by ALC (average of local confidence) score. ALC is the average of the local confidence score of all the amino acids in the sequence. ALC >55% is the minimum manufacturer recommended value to consider an acceptable matching for *de novo* peptide match. For *the novo* sequencing on analysed samples, a previous data refinement was done taking into account the molecular weight cut-off of each fraction. A mass error of 10 ppm on precursor mass and 0.8 Da in fragment ions were allowed and oxidation of methionine residues was selected as a possible modification.

Study of the antihypertensive activity in spontaneously hypertensive rats (SHR)

The antihypertensive effect of the peptides of the invention was studied on the blood pressure of spontaneously hypertensive rats. The development of high blood pressure (hypertension, HT) in SHR has clear analogies with the development of HT in humans. The peptides were purified *ad hoc* for these studies. Systolic blood pressure (SBP) and diastolic blood pressure (DBP) of rats was measured using the "tail cuff" method. This model is not invasive and the only contact with the animals is the careful administration of a small volume of the extract of the study, followed by the determinations of the SBP and DBP. Before positioning the cuff and transducer in the tail of the rats, the animals were exposed to a temperature close to 37ºC to facilitate dilation of the caudal artery. To avoid interferences and since SHR are especially nervous, the animals were familiarised with the procedure for a period of time of 2 to 3 weeks before the beginning of the experiment.
Three consecutive measurements were taken of SBP and DBP from each animal. Average values were used. The animals used were male SHR, 17-20 weeks old, weighing about 0.3 kg. For the test, the SHR were kept at of 23°C, with light-dark cycles of 12 hours. The animals had free access to water and food (standard chow diet). The rats were randomly divided into 3 study groups. The animals received an oral dose of 5 ml/kg of the following compounds:

- Negative control group (n = 8): water.
- Positive control group (n = 8): Captopril at a dose of 50 mg/Kg
- Experimental group 1 (n = 8): Olive oil concentrated peptides at a dose of 0.425 mg/Kg

SBP and DBP was measured in the rats before administration of the corresponding dose and 2, 4, 6, 8, 24 and 48 hours later. Data were analysed using one-way ANOVA with SPSS 15.0 software. P values<0.05 were considered significantly different. [M Quinones, Miguel M, Muguerza B, Aleixandre A. Effect of a cocoa polyphenol extract in spontaneously hypertensive rats. Food Funct. 2011; 649-653]

**EXAMPLE 1: Preparation of an anti-hypertensive composition**

Olive oil is obtained from olives of the Picual variety. The olives were harvested directly from the trees at the turning stage of ripening. The olives were washed and the olive oil was extracted using a two-phase extraction plant which produces olive oil, olive pomace and olive mill wastewater. The extraction of the olive oil was carried out within 48 hours of collection of the olives. A first extraction of the peptides of the invention was performed in several batches with a mixture of acetone:hexane (1:1) using an olive oil/solvent ratio of 1:2.5 (w/v), for 1 h in the cold room at 4-6°C. Then, the mixture was centrifuged at 10,000 g for 15 minutes at 4°C. The supernatant was discarded and the precipitate, containing the peptides, was separated. The extraction process was repeated with each olive oil batch. The precipitates containing the peptides were pooled and desiccated (initial extract), always maintaining a temperature below 40°C. Next, the initial extract was suspended in suitable mixtures of water and acetonitrile, preferably 80:20. Then, the extract underwent a process of sonication for 30 minutes and it was centrifuged again at 10,000 g for 15 minutes at 4°C. The supernatants, containing the concentrated olive oil peptides were collected. This concentrate possesses ACE inhibitory activity (see Example 2).

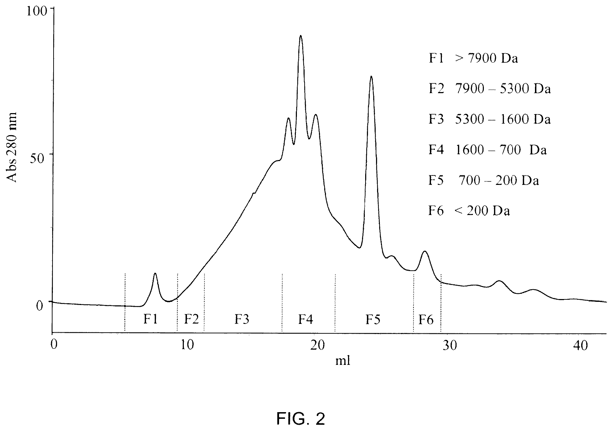

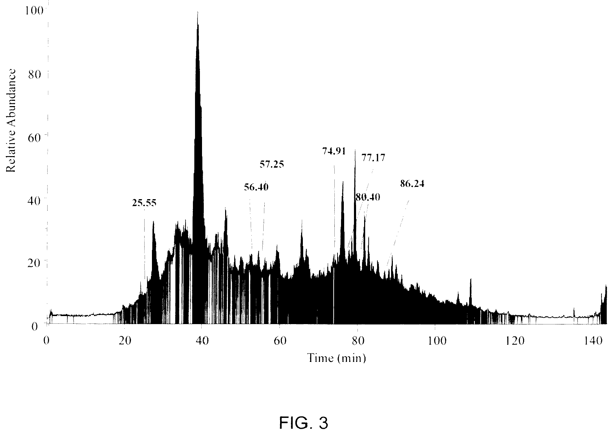

The concentrated olive oil peptides can be purified with an FPLC system using a molecular exclusion column Superdex Peptide 10/300 GL (GE Healthcare, UK) capable of separating peptides between 100 and 7000 Da. Three major peptide fractions were selected, with molecular masses ranging from 5300 to 1600 Da (F3), 1600-700 Da (F4) and 700-200 Da (F5, see Figure 2). The olive oil concentrates peptides of the invention (before purification by FPLC), and the peptides contained in the FPLC purified fractions, were analysed by nanoLC-Orbitap-MS / MS (Figure 3), in triplicate, and their amino acid sequences were obtained by *de novo* sequencing as described in the analytical methods section. Several new peptides with angiotensin-converting enzyme inhibitory activity were identified, with the sequence numbers SEQ ID NO: 1, SEQ ID NO: 2, SEQ ID NO: 3, SEQ ID NO: 4, SEQ ID NO: 5, SEQ ID NO: 6, SEQ ID NO: 7 (see Table 2).

Table 2: New peptides with angiotensin-converting enzyme inhibitory activity and their retention time.

| Sequence number | Peptide sequence | Retention time (min) |
|---|---|---|
| SEQ ID N°1 | RDGGYCC | 57,25 |
| SEQ ID N°2 | YYCPADCPS | 77,17 |
| SEQ ID N°3 | YGCGCDPL | 86,24 |
| SEQ ID N°4 | LEEFCC | 80,40 |
| SEQ ID N°5 | HCGCNTH | 56,40 |
| SEQ ID N°6 | WAAGYCC | 74,91 |
| SEQ ID N°7 | CCGNAVPQ | 25,55 |

Olive oil concentrated peptides are subjected to a drying process, preferably spray drying, after the addition of maltodextrin (to reach a minimum 2% of total solids), resulting in a dried concentrated extract containing the peptides of the invention. Alternative drying methods are evaporation, lyophilisation or others. The dried concentrated extract (powder) can be used as such, dissolved in water, aqueous solution, gelatine, organic solvents or mixtures thereof, for food and pharmaceutical applications.

**EXAMPLE 2: Inhibition of angiotensin-converting enzyme**

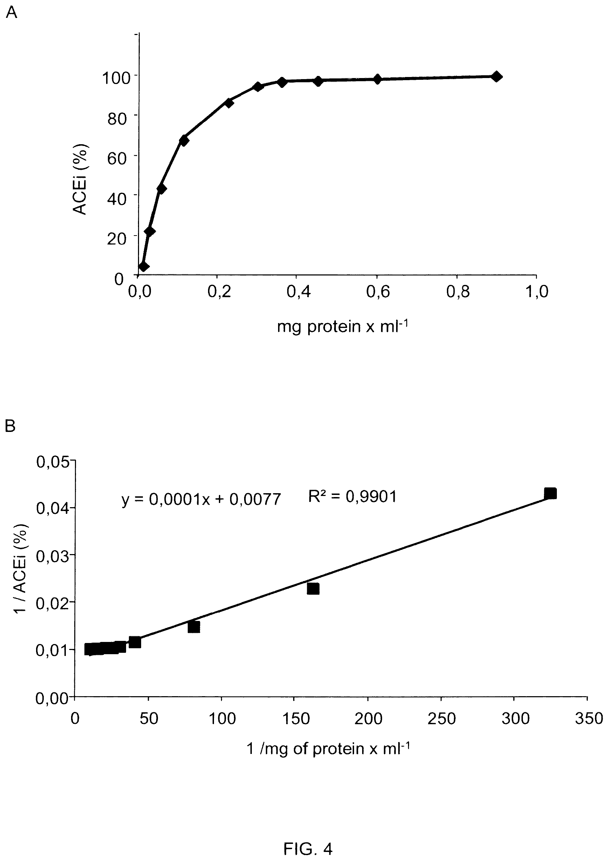

ACE inhibitory activity was determined for olive oil concentrated peptides and also for the three FPLC purified fractions F3, F4 and F5 described in example 1 (see Table 3 and Figure 4).

Table 3. ACE inhibitory activity of olive oil concentrated peptides and of the FPLC-purified fractions F3, F4 and F5. Data are expressed as mean values $\pm$ standard deviation.

| Sample | $IC_{50}$ ($\mu$g/ml) |
|---|---|
| Olive oil concentrated peptides | 2,5$\pm$0,2 |
| FPLC purified peptides fraction F3 (5300-1600 Da) | 47,6$\pm$2,4 |
| FPLC purified peptides fraction F4 (1600-700 Da) | 154,0$\pm$0,9 |
| FPLC purified peptides fraction F5 (700-200 Da) | 98,0$\pm$5,0 |

The olive oil peptides that showed the highest abundance were selected and chemically synthesized, more specifically SEQ ID NO: 1, SEQ ID NO: 2, SEQ ID NO: 3, SEQ ID NO: 4, SEQ ID NO: 5, SEQ ID NO: 6 and SEQ ID NO: 7. ACE inhibitory activity was determined for these synthesized peptides (purity range 95-100%), as shown in Table 4.

**Table 4.** ACE inhibitory activity of selected olive oil peptides obtained by synthesis (purity range 95-100%). Data are expressed as mean values ± standard deviation.

| Sequence number | Peptide sequence | $IC_{50}$ (µM) |
|---|---|---|
| SEQ ID Nº1 | RDGGYCC | 0,84±0,02 |
| SEQ ID Nº2 | YYCPADCPS | 1,64±0,02 |
| SEQ ID Nº3 | YGCGCDPL | 1,85±0,16 |
| SEQ ID Nº4 | LEEFCC | 2,58±0,26 |
| SEQ ID Nº5 | HCGCNTH | 2,59±0,22 |
| SEQ ID Nº6 | WAAGYCC | 6,88±0,30 |
| SEQ ID Nº7 | CCGNAVPQ | 39,56±2,09 |

The olive oil concentrated peptides, the 3 major FPLC-purified fractions (F3-F5) and the individual peptides obtained by synthesis showed significant ACE inhibitory activity. Therefore, we conclude that inhibition of ACE is the mechanism of action of the antihypertensive activity of the olive oil peptides. However, other additional mechanisms cannot be ruled out.

**EXAMPLE 3: Blood-pressure lowering effect in hypertensive rats**

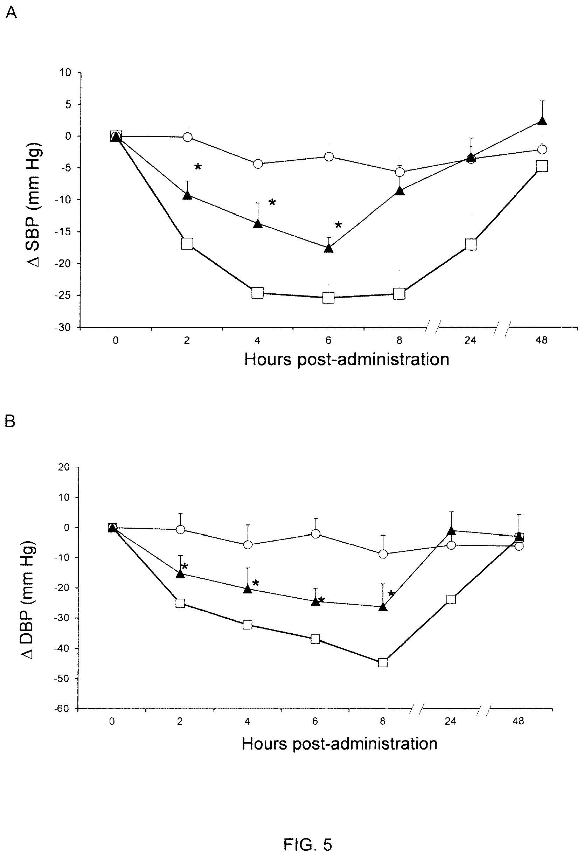

The antihypertensive activity of olive oil concentrated peptides was studied in a model of spontaneously hypertensive rats (SHR). Figure 5 shows changes in systolic blood pressure (SBP) and diastolic blood pressure (DBP) obtained in SHR after administration of the different compounds tested. The initial values of SBP and DBP of the SHR before the administration of the test solutions were 210 ± 2.3 mmHg and 157 ± 3.0 mm Hg, respectively. Captopril produced a very sharp decline in SBP and DBP in SHR. The maximal hypotensive effect was registered between 4 and 6 hours after the administration. SBP and DBP returned to baseline values after 48 hours.
The olive oil concentrated peptides produced significant reductions in SBP and DBP in SHR. In the case of SBP, reduction began to be detected 2 hours after the administration of the extract and it was measurable for the first 6 h, before returning to baseline. In the case of the DBP, the olive oil concentrated peptides also produced reductions that were detectable at 2, 4, 6 and 8 hours after the administration. The data of SBP and DBP obtained at 48 hours were similar to those obtained at 24 hours. In conclusion, the composition of olive oil peptides described in example 1 possess antihypertensive activity.

**EXAMPLE 4: Preparation of a foodstuff containing an anti-hypertensive composition**

An enriched juice was prepared using the following ingredients:

| Ingredient | Amounts per Kg |
|---|---|
| Concentrated juice | 200 g |
| Dry extract containing the olive oil peptides of the invention | 1 g |
| Insoluble fibre | 10 g |
| Lecithin | 0.5 g |
| Flavors | 2 g |
| Vitamin C | 90 mg |

(continued)

| Ingredient | Amounts per Kg |
|---|---|
| Vitamin B1 | 2.1 mg |
| Vitamin B2 | 2.4 mg |
| Vitamin B6 | 3 mg |
| Vitamin B12 | 1.5 μg |
| Vitamin A | 1.2 mg |
| Vitamin D | 7.5 μg |
| Folic acid | 300 μg |
| Water | 786.4 g |

Processing technology:

The final product was prepared from a concentrated juice by addition of water and water soluble ingredients. Then, the dried extract containing the olive oil peptides of the invention having the sequences SEQ ID NO: 1, SEQ ID NO: 2, SEQ ID NO: 3, SEQ ID NO: 4, SEQ ID NO: 5, SEQ ID NO: 6 and SEQ ID NO: 7 was added and mixed and the resulting product was pasteurized and homogenized. Finally, the product was cooled and packaged.

**EXAMPLE 5: Preparation of an oil-based food (margarine) containing an anti-hypertensive composition**

An enriched spreadable (margarine) may be prepared using the following ingredients:

| Ingredient | Amounts per Kg | |
|---|---|---|
| Fat phase: | | |
| Fat oil | 700 | g |
| Refined sunflower oil | 275 | g |
| Deodorised and refined fish oil | 15 | g |
| Emulsifiers (mono- and diglycerides of fatty acids, and lecithin) | 10 | g |
| Vitamin A | 2 | mg |
| Vitamin D | 18,75 | μg |
| Vitamin E | 50 | mg |
| Aqueous phase | | |
| Water | 956,3 | g |
| Dry extract containing the olive oil peptides of the invention | 16,6 | g |
| Flavors | 2 | |
| Sodium alginate | 16,6 | g |
| Salt | 6 | g |
| Potassium sorbate | 1,6 | g |
| Lactic acid | 0,6 | g |
| Vitamin C | 150 | mg |
| Vitamin B1 | 3,5 | mg |
| Vitamin B2 | 4 | mg |
| Vitamin B6 | 5 | mg |

(continued)

| Aqueous phase | | |
|---|---|---|
| Vitamin B12 | 2,5 | μg |
| Folic acid | 500 | μg |

Processing technology:

The spread is prepared using a similar process used for margarine production. A water-in-oil emulsion is prepared by dispersing the aqueous phase in the fat phase in the proportion of 60% aqueous phase to 40% fat phase by weight of the water-in-oil emulsion.

To prepare the fat phase, ingredients are added to a mixing tank, the temperature is increased to 45ºC and the ingredients are mixed until complete homogeneity. The ingredients of the aqueous phase are mixed in a separate tank at 75°C. With the oil phase at 45°C and the aqueous phase at 75°C, the aqueous phase in the proportion 60% by weight of the water-in-oil emulsion is dispersed in the fat phase in the proportion of 40% by weight of the water-in-oil emulsion to form the water-in-oil emulsion. The resulting water-in-oil emulsion is processed by conventional means, typically, in a scraped surface exchanger and ancillary equipment for pasteurization and is then cooled. The emulsion is cooled and subjected to a texturing process for providing spreadable consistency so it is solid at room temperature.

**EXAMPLE 6: Preparation of a yogurt containing an anti-hypertensive composition**

Yogurt and fermented milks can be prepared using the following ingredients:

| Ingredient | Amounts per Kg | |
|---|---|---|
| Milk containing 3.1% fat and 3.2% protein | 966,9 | g |
| Skimmed milk powder | 13 | g |
| Dry extract containing the olive oil peptides of the invention | 20 | g |
| Yogurt starter culture | 0,1 | g |

Processing technology:

The milk fat and milk solids content are standardized according to the formula above and the peptides of the invention are added. The milk is homogenised at 20-25 MPa, at a temperature of 65-70°C. Next, the milk is heated at 90-95°C for 5 minutes. This treatment is able to denature about 70-80% of whey proteins. Then the milk is cooled to 40-45°C and yogurt starter culture is dispensed during the transportation of the milk from the storage tank to the filling machine. Once the filling phase of the product has ended, it is then transported to an incubation chamber where fermentation takes place at 40-45°C for 5-6 hours, or until the pH value reaches 4.5. Finally, the resulting fermented milk is stored in a chamber at 5°C.

**EXAMPLE 7: Preparation of gelatin capsules containing an anti-hypertensive composition**

| Substance | Amounts per Kg | |
|---|---|---|
| Dry extract containing the olive oil peptides of the invention | 1,5 | g |
| Anhydrous lactose | 42,6 | g |
| Magnesium stearate | 0,9 | g |
| Hard gelatine capsules # 2 | 150 | cápsulas |

Processing technology:

Raw materials are weighed and transferred to the manufacturing area. The anhydrous lactose and magnesium stearate are sieved. 50% (w/w) of the anhydrous lactose is added to the magnesium stearate and mixed for 5 minutes in a mixer, at medium speed. Next, the dry extract containing the olive oil peptides of the invention is added and further mixed for 5 minutes. The remaining 50% (w/w) of the anhydrous lactose is then added and mixed again. The resulting product is encapsulated in hard gelatine capsules of 300 mg using a manual, semiautomatic or automatic capsule filling system, according to established pharmaceutical procedures.

**EXAMPLE 8: Preparation of an edible oil blend containing an anti-hypertensive composition**

Olive oil or any type of edible oil may be enriched with the peptides of the invention using the following ingredients:

| Ingredient | Amounts per Kg | |
|---|---|---|
| Fat phase: | | |
| Olive oil | 931,4 | g |
| lecithin | 2 | g |
| Aqueous phase: | | |
| Water | 50 | g |
| Dry extract containing the olive oil peptides of the invention | 16,6 | g |

Processing technology:

An emulsion of water-in-oil (W/O emulsion) is prepared by dispersing the aqueous phase in the oil phase. For this, the oil phase (olive oil or any edible oil) is heated to 45°C. Then olive oil and lecithin are mixed until complete homogeneity. Next, the dried extract containing the olive oil concentrated peptides of the invention, is dissolved in water at 70ºC. Finally, the aqueous phase is added to the oil phase and they are gently mixed until complete homogeneity. The resulting emulsion is heated before packaging as it may have a cloudy appearance.

**Claims**

1. An isolated peptide, or any of salts, esters, solvates and anhydrates pharmaceutically acceptable thereof, comprising at least two cysteines separated by 0 amino acid, consisting of a length between 8, 9 or 10 amino acids, wherein the peptide shows anti-hypertensive activity and comprises the sequence SEQ ID NO: 7.

2. A fusion peptide comprising the peptide according to claim 1 and a carrier or marker peptide, wherein

   - the carrier peptide is a peptide which internalizes a peptide in the cell, and
   - the marker peptide is an amino acid sequence useful for the isolation or purification of the isolated peptide.

3. A peptide according to claim 1 or 2, for use as a medicament.

4. A peptide according to claim 1 or 2, for use in the treatment and/or prevention of pre-hypertension or hypertension.

5. A method for obtaining a peptide according to claim 1 or 2 comprising

   (i) obtaining a protein precipitate from the oil coming from olives,
   (ii) obtaining a peptide extract from the precipitate of step (i) comprising the peptide, and
   (iii) Fractionating and purifying the peptide according to claim 1 or 2.

6. Method according to claim 5, wherein the oil comes from mashing and/or milling of olives, preferably, the oil comes from the group consisting of crushed olives paste, pomace oil, olive pomace, olive mill wastewater, dried/defatted olive pomace cake, and blends and mixtures thereof.

**Patentansprüche**

1. Isoliertes Peptid oder pharmazeutisch verträgliche Salze, Ester, Solvate und Anhydrate davon, umfassend mindestens zwei durch 0 Aminosäuren getrennte Cysteine, bestehend aus einer Länge zwischen 8, 9 oder 10 Aminosäuren, wobei das Peptid eine antihypertensive Aktivität zeigt und die Sequenz SEQ-ID-NO: 7 umfasst.

2. Fusionspeptid, umfassend das Peptid nach Anspruch 1 und ein Träger- oder Markerpeptid, wobei

   - das Trägerpeptid ein Peptid ist, das ein Peptid in der Zelle internalisiert, und
   - das Markerpeptid eine Aminosäuresequenz ist, die für die Isolierung oder Reinigung des isolierten Peptids nützlich ist.

3. Peptid nach Anspruch 1 oder 2 zur Verwendung als Medikament.

4. Peptid nach Anspruch 1 oder 2 zur Verwendung bei der Behandlung und/oder Vorbeugung von Prähypertonie oder Hypertonie.

5. Verfahren zum Erhalten eines Peptids nach Anspruch 1 oder 2, umfassend

   (i) Gewinnen eines Proteinniederschlags aus dem von Oliven stammenden Öl,
   (ii) Gewinnen eines Peptidextrakts aus dem Niederschlag von Schritt (i), umfassend das Peptid, und
   (iii) Fraktionieren und Reinigen des Peptids nach Anspruch 1 oder 2.

6. Verfahren nach Anspruch 5, wobei das Öl aus dem Maischen und/oder Mahlen von Oliven stammt, wobei das Öl vorzugsweise aus der Gruppe, bestehend aus zerkleinerter Olivenpaste, Tresteröl, Oliventrester, Olivenmühlenabwasser, getrocknetem/entfettetem Oliventresterkuchen sowie Gemischen und Mischungen davon, stammt.

**Revendications**

1. Peptide isolé, ou l'un quelconque des sels, esters, solvates et anhydrates pharmaceutiquement acceptables de celui-ci, comprenant au moins deux cystéines séparées par 0 acide aminé, constitué d'une longueur comprise entre 8, 9 ou 10 acides aminés, dans lequel le peptide montre une activité anti-hypertensive et comprend la séquence SEQ ID NO : 7.

2. Peptide de fusion comprenant le peptide selon la revendication 1 et un peptide vecteur ou marqueur, dans lequel

   - le peptide vecteur est un peptide qui internalise un peptide dans la cellule, et
   - le peptide marqueur est une séquence d'acides aminés utile pour l'isolement ou la purification du peptide isolé.

3. Procédé selon la revendication 1 ou 2, destiné à être utilisé comme médicament.

4. Procédé selon la revendication 1 ou 2, destiné à être utilisé dans le traitement et/ou la prévention de la pré-hypertension ou de l'hypertension.

5. Procédé d'obtention d'un peptide selon la revendication 1 ou 2 comprenant

   (i) l'obtention d'un précipité protéique à partir de l'huile provenant d'olives,
   (ii) l'obtention d'un extrait peptidique à partir du précipité de l'étape (i) comprenant le peptide, et
   (iii) le fractionnement et la purification du peptide selon la revendication 1 ou 2.

6. Procédé selon la revendication 5, dans lequel l'huile provient de la transformation en purée et/ou du broyage d'olives, de préférence, l'huile provient du groupe constitué par la pâte d'olives concassées, l'huile de grignons, les grignons d'olive, les eaux usées des moulins à huile, un gâteau de grignons d'olive séchés/dégraissés, et des assemblages et des mélanges de ceux-ci.

Olives (*Olea europaea* L)

Crushed olive paste

Olive pomace +
Olive mill wastewater          Olive oil

Olive pomace          Olive mill
wastewater

Olive pomace oil          Olive pomace cake
(defatted and dehydrated)

FIG. 1

F1 > 7900 Da

F2 7900 – 5300 Da

F3 5300 – 1600 Da

F4 1600 – 700 Da

F5 700 – 200 Da

F6 < 200 Da

FIG. 2

FIG. 3

A

B

FIG. 4

A

B

FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1568707 A1
- EP 2495250 A2

- US 4477440 A

**Non-patent literature cited in the description**

- **SAADI S ; SAARI N ; F ANWAR ; ABDUL HAMID A ; HM GHAZALI.** Recent advances in food biopeptides: Production, biological functionalities and therapeutic applications. *Biotechnol Adv.,* 2015, vol. 33, 80-116
- **MANDAL SM ; BHARTI R ; PORTO WF ; SS NECESSARY GAURI ; MANDAL M ; FRANCO OL ; AK GHOSH.** Identification of multifunctional peptides from human milk. *Peptides,* 2014, vol. 56, 84-93
- **RICCI I ; ARTACHO R ; M. OLALLA.** With Milk protein peptides angiotensin I-converting enzyme inhibitory (ACEI) activity. *Crit Rev Food Sci Nutr.,* 2010, vol. 50, 390-402
- **ESTEVE C. et al.** *Food Chemistry,* 2015, vol. 167, 272-280
- **ALTSCHUL et al.** *J. Mol. Biol.,* 1990, vol. 215, 403-410
- **SCHWARZE S.R. et al.** *Science,* 03 September 1999, vol. 285 (5433), 1569-72
- **NIESNER U. et al.** *Bioconjug. Chem.,* July 2002, vol. 13 (4), 729-36

- **FORD K.G. et al.** *Gene Therapy,* 2001, vol. 8, 1-4
- **GUSAROVA G.A. et al.** *J. Clin. Invest.,* January 2007, vol. 117 (1), 99-111
- **TERPE K.** *Appl. Microbiol. Biotechnol.,* 2003, vol. 60, 523-525
- **SENTANDREU, M.A. ; TOLDRÁ, F.** A fluorescence-based protocol for quantifying angiotensin-converting enzyme activity. *Nat. Protoc.,* 2006, vol. 1 (5), 2423-2427
- **SIN MA ; KAIZHONG ZHANG ; CHRISTOPHER HENDRIE ; CHENGZHI LIANG ; MING LI ; AMANDA DOHERTY-KIRBY ; GILLES LAJOIE.** PEAKS: Powerful Software for Peptide De Novo Sequencing by MS/MS. *Rapid Communications in Mass Spectrometry,* 2003, vol. 17 (20), 2337-2342
- **M QUINONES ; MIGUEL M ; MUGUERZA B ; ALEIXANDRE A.** Effect of a cocoa polyphenol extract in spontaneously hypertensive rats. *Food Funct.,* 2011, 649-653